Europäisches Patentamt

European Patent Office

Office européen des brevets

(19)

(11) Numéro de publication : **0 439 405 A1**

# (12) DEMANDE DE BREVET EUROPEEN

(21) Numéro de dépôt : **91400150.8**

(22) Date de dépôt : **23.01.91**

(51) Int. Cl.⁵ : **G01N 3/42,** G01N 33/02, G01B 3/00

(30) Priorité : **23.01.90 FR 9000756**

(43) Date de publication de la demande :
**31.07.91 Bulletin 91/31**

(84) Etats contractants désignés :
**DE ES GB IT NL**

(71) Demandeur : **INSTITUT NATIONAL DE LA RECHERCHE AGRONOMIQUE (I.N.R.A.) 147, rue de l'Université F-75341 Paris Cédéx 01 (FR)**
Demandeur : **CENTRE TECHNIQUE INTERPROFESSIONNEL DES FRUITS ET LEGUMES (C.T.I.F.L.) 6, rue de Rougemont F-75009 Paris (FR)**

(72) Inventeur : **Abbal, Philippe
722, Chemin de l'aire des masques
F-34230 Montferrier sur Lez (FR)**
Inventeur : **Planton, Gérard
11, rue de la Source
F-30230 Bouillargues (FR)**

(74) Mandataire : **Phélip, Bruno et al
c/o Cabinet Harlé & Phélip 21, rue de La Rochefoucauld
F-75009 Paris (FR)**

(54) **Dispositif pour déterminer automatiquement des caractéristiques physiques d'un produit et procédé de mesure mis en oeuvre au moyen de ce dispositif.**

(57)    Le dispositif comprend des premiers moyens de support (1) d'une tige de mesure (2) dont une extrémité (2a) est conçue pour supporter un embout de travail (3) destiné à venir en contact avec le produit (10).

Lesdits premiers moyens de support (1) sont montés à translation par rapport à une vis sans fin (4) qui s'étend parallèlement à la tige de mesure (2) et qui est entraînée en rotation par des moyens moteurs (5), tandis que le produit est disposé sur des seconds moyens de support (6) formant capteurs de mesure qui sont reliés à des moyens informatiques (8) programmés pour traiter et stocker les mesures réalisées par le dispositif.

Application à l'évaluation de la fermeté des fruits et des légumes ou de tout autre objet solide comparable.

EP 0 439 405 A1

FIG.1

# DISPOSITIF POUR DETERMINER AUTOMATIQUEMENT DES CARACTERISTIQUES PHYSIQUES D'UN PRODUIT ET PROCEDE DE MESURE MIS EN OEUVRE AU MOYEN DE CE DISPOSITIF

La présente invention concerne un dispositif pour déterminer automatiquement des caractéristiques physiques telles que les caractéristiques dimensionnelles, la masse et/ou la fermeté d'un produit, notamment un fruit ou un légume.

L'invention vise également un procédé de mesure desdites caractéristiques mises en oeuvre au moyen du dispositif précité.

Il n'existe pas actuellement d'appareil permettant de déterminer de façon fiable, précise, fidèle et répétitive, la fermeté de produits tels que des fruits et des légumes. Les appareils couramment utilisés, en particulier dans les laboratoires de recherche, sont des dynamomètres manuels dont la précision est très affectée par l'intervention d'un opérateur. Or, la fermeté est un critère très important dans l'appréciation de la qualité d'un produit.

Le présent déposant a déjà mis au point un appareil du genre décrit au début du présent mémoire, dont les caractéristiques sont récitées dans le FR-A-2535824. Cette construction originale est toutefois difficile à réaliser à l'échelle industrielle. Le FR-A-2585833 et le FR-A-2560683 décrivent également des dispositifs du genre précité dont l'utilisation est toutefois limitée à des mesures très classiques et peu variées.

L'existence d'une référence en matière de fermeté de produits tels que des fruits et légumes devient indispensable car de nombreuses applications de recherche et développement sont attendues : comparaisons de variétés, de techniques culturales, de méthodes de conservation et d'emballage, évolution des produits dans les circuits de distribution et de mise en marché.

Partant d'une analyse initiale et de la réalisation décrite par le FR-A-2595824 précité, le but de la présente invention est de proposer un appareil de mesure fiable, simple et précis qui présente en outre un bon rapport qualité-prix et qui soit facile à assembler en faisant appel à un choix judicieux d'éléments standards disponibles dans le commerce. En particulier, l'invention vise à proposer un ensemble de mesures architecturées autour d'un calculateur informatique utilisant un logiciel dont le rôle est double, c'est-à-dire à la fois de piloter la machine de façon à lui faire exécuter des séquences de travail adaptées au produit testé, telles que par exemple une pénétration dans des produits durs, un écrasement de produit mou, un arrachement de pédoncule, et également de recueillir les données obtenues, de les stocker et de les traiter de manière automatique.

Ces moyens sont combinés dans la présente invention avec un dispositif mécanique qui permet une grande précision de mesure, et notamment de mesure des mensurations des produits analysés. Il est à noter que le système à engrenage et à crémaillère du dispositif de la demande FR-A-2595824 était, quant à lui, peu précis et susceptible de prendre facilement du jeu, alors que le système proposé par l'invention permet une analyse des mensurations des produits au dixième de millimètre.

Le dispositif mécanique que propose l'invention en combinaison avec les moyens de mesure précités comporte une tige de mesure supportant un embout de travail destiné à venir en contact avec le produit, ladite tige de mesure étant montée en translation par rapport à une vis sans fin qui lui est parallèle.

Il existe un dispositif décrit dans DE-3219766 qui a été mis au point dans un domaine technique totalement différent de celui de l'invention, étant donné qu'il s'agit d'un dispositif de guidage d'une machine outil pour le polissage de pièces métalliques. L'invention concerne, quant à elle, un dispositif permettant la description physique de produits vivants, tels que des fruits et des légumes, dont on souhaite déterminer les mensurations (poids, diamètre) et la fermeté. De ce fait, le dispositif de l'invention est destiné à travailler sur des produits de dimensions très différentes, alors que le polissage réalisé par le dispositif de DE-3219766 se fait à des cotes définies à l'avance. En outre, le dispositif de l'invention est destiné à travailler sur des produits indifféremment durs et mous, alors que les pièces polissées par le dispositif de DE-3219766 sont nécessairement dures. Le dispositif de DE-3219766 ne comporte d'ailleurs aucun capteur de fermeté. Accessoirement, il faut également noter que les moyens décrits dans DE-3219766 ne permettent aucun stockage de résultats analogues à celui que permet l'invention.

La présente invention a donc pour objet un dispositif conçu pour déterminer automatiquement des caractéristiques physiques telles que les caractéristiques dimensionnelles, la masse et/ou la fermeté d'un produit, notamment un fruit ou un légume, est caractérisé par des premiers moyens de support d'une tige de mesure dont une extrémité est conçue pour supporter un embout de travail destiné à venir en contact avec le produit, lesdits premiers moyens de support de la tige de mesure étant monté à translation par rapport à une vis sans fin qui s'étend sensiblement parallèlement à la tige de mesure et qui est entraînée en rotation par des moyens moteurs du dispositif, et en ce que ledit produit est disposé sur des seconds moyens de support formant capteur de mesure dont le plan de travail s'étend sensiblement perpendiculairement à la tige de mesure, les moyens formant capteur de mesure étant reliés à des moyens informatiques du type ordinateur ou analogue qui sont également reliés à des moyens de commande électroniques du moteur, lesdits moyens informatiques étant programmés pour traiter et stocker les mesures réalisées par le dispositif.

Selon certaines caractéristiques préférentielles de l'invention, lesdits moyens moteurs sont reliés aux moyens informatiques par l'intermédiaire d'un circuit électronique de pilotage à micro-contrôleur qui réalise le dialogue avec les moyens informatiques, ce dialogue entre le circuit de pilotage et les moyens informatiques étant réalisé par l'intermédiaire d'un port série à 9600 bits par seconde du circuit de pilotage, la tension de sortie analogique délivrée par les moyens capteurs est appliquée aux moyens informatiques par l'intermédiaire d'un circuit d'interface conçu pour assurer une conversion analogique digitale de cette tension de sortie et pour détecter rapidement toute variation de mesure, et les moyens moteurs comprennent un moteur pas à pas dont l'arbre de sortie est couplé à ladite vis sans fin par l'intermédiaire d'un moto-réducteur qui produit une avance d'environ 200 pas par tour moteur.

Selon d'autres caractéristiques préférentielles de l'invention, les moyens formant capteur comprennent une balance de précision de type METTLER qui présente une sensibilité d'environ 0,1 gramme et qui possède un port série ainsi qu'une sortie analogique reliés aux moyens informatiques tandis que les premiers moyens de support comprennent un chariot monté sur un portique-support par l'intermédiaire d'un dispositif à double guidage et entraîné par la vis sans fin par l'intermédiaire de vis à billes.

Par ailleurs, chaque embout de travail comporte, du côté de la tige de mesure, un culot de fixation amovible à l'extrémité de cette tige de mesure qui est conformée en mandrin de perceuse et, du côté opposé, une tige de pénétration du produit ou un plateau d'écrasement de celui-ci. Le dispositif selon l'invention est en outre caractérisé par au moins un support en aluminium de positionnement du produit sur le plan de travail des moyens formant capteurs.

L'invention propose également un procédé de mesure mis en oeuvre au moyen du dispositif précité, caractérisé par les étapes consistant à :

a) descendre l'embout de travail jusqu'à détection par le port analogique des moyens formant capteur d'une tension différentielle indicative de la présence d'un produit sur le plan de travail des moyens capteurs,

b) déduire, à partir du nombre de tours effectués par les moyens moteurs, la hauteur du produit

c) faire l'acquisition de la masse du produit au moyen de la liaison série entre les moyens capteurs et les moyens informatiques

d) stocker les paramètres de mesure précités sur un fichier disque et, sur ce même fichier, des références de l'échantillon de produit.

Le procédé précité est en outre caractérisé par les étapes complémentaires consistant à :

– saisir la profondeur de pénétration du produit souhaitée, cette profondeur étant une valeur fixée ou bien une valeur calculée par l'ordinateur et dépendant de la hauteur du produit (pourcentage),

– choisir la vitesse de pénétration,

– répéter les étapes a) à d) précitées,

– déduire de la profondeur souhaitée, le nombre de pas moteur à parcourir,

– exécuter l'ordre de mesure,

– stocker la force maximale à appliquer et la profondeur d'obtention de cette force ainsi que les points intermédiaires (par exemple 10) permettant de tracer la courbe force appliquée/profondeur,

– répéter si nécessaire les étapes précédentes.

Selon une variante de l'invention, le procédé en question est caractérisé par les étapes complémentaires consistant à :

– saisir le type d'écrasement souhaité en hauteur fixée, en pourcentage de hauteur du produit, ou en masse à appliquer,

– répéter les étapes a) ou d) précitées,

– en déduire le nombre de pas moteur,

– exécuter l'ordre de mesure,

– stocker la force maximale appliquée et sa profondeur d'obtention ainsi que 10 points intermédiaires ou, selon le cas,

– calculer la hauteur parcourue par l'embout d'après le nombre de pas moteur jusqu'à obtention de la masse fixée et stocker cette hauteur, et

– répéter si nécessaire les étapes précitées.

Convenablement interfacés, les sous-ensembles décrits précédemment permettent, grâce à un logiciel convivial écrit en langage TURBO C (Borland) de déterminer avec précision les principaux éléments de la fermeté. L'ensemble des résultats est préférentiellement stocké sur un ou plusieurs supports magnétiques afin de permettre l'analyse des données recueillies.

La conception du dispositif conforme à l'invention que l'on appellera dans la suite de la description pénétromètre, rend ce dispositif essentiellement évolutif tant par le matériel choisi que par le logiciel. Les mesures suivantes peuvent être réalisées à l'aide de ce pénétromètre :

– mensurations et pesées,

– force maximale pour réaliser la pénétration d'un embout défini d'une hauteur déterminée H dans un produit dur (de type pomme) ou en pourcentage de la hauteur du produit,

– profondeur nécessaire à l'obtention de cette force maximale,

– 10 points intermédiaires permettant le tracé force/pénétration,

– écrasement d'un produit mou (type cerise) sous une force donnée, % de hauteur donnée ou profondeur fixée,

– force maximale recueillie lors de l'écrasement d'une hauteur fixée ou d'un pourcentage connu de la hauteur initiale d'un produit mou,

– possibilité d'associer la masse du produit à sa fermeté,

– possibilité d'écraser plusieurs fois le même produit au même endroit pour tester son élasticité (rhéologie),

– possibilité d'effectuer sur le même produit plusieurs mesures en différents points afin d'en obtenir une valeur moyenne tenant compte de la variabilité biologique,

– force d'arrachement des pédoncules de cerise (application à la récolte mécanique).

Le pénétromètre selon l'invention présente également l'avantage de difficilement se détériorer malgré la force importante développée par sa vis sans fin.

D'autres caractéristiques et' avantages de l'invention ressortiront encore de la description qui va suivre.

Aux dessins annexés donnés à titre d'exemples non limitatifs :

La Figure 1 est une vue schématique de côté du pénétromètre conforme à l'invention, qui comprend également un schéma-bloc des moyens électroniques mis en oeuvre,

La Figure 2 est une vue de face du pénétromètre de la Figure 1,

La Figure 3 est une vue de côté d'un élément support utilisé pour placer le produit sur les moyens formant capteur,

Les Figures 4A et 4B sont des vues de côté des embouts utilisés, et

La Figure 5 est une vue du dispositif d'arrachement des pédoncules de cerise.

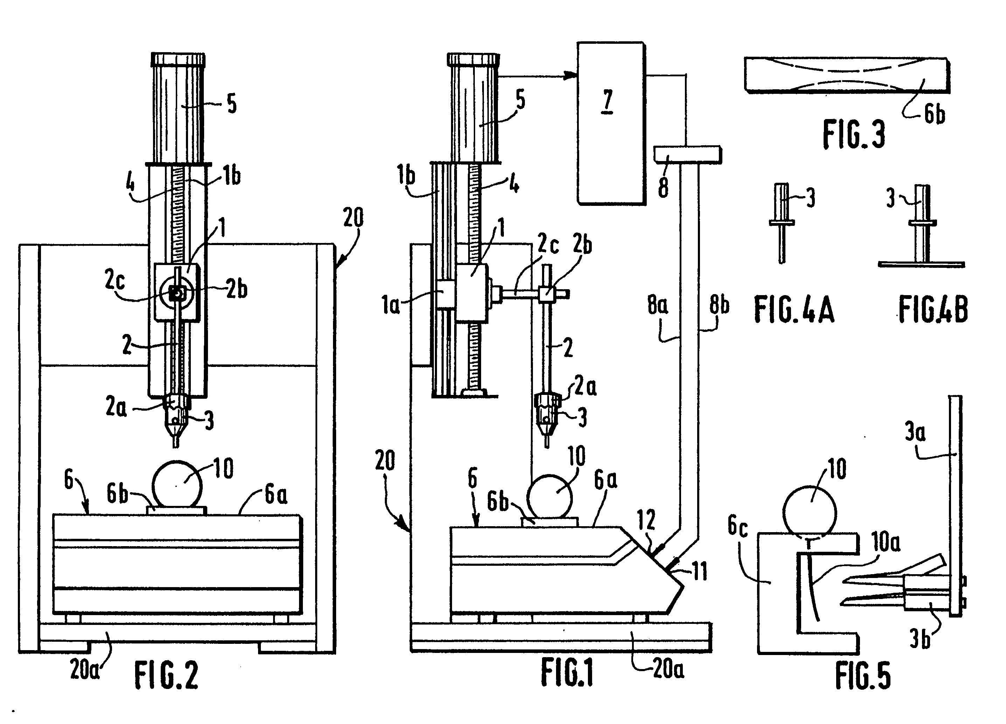

Le pénétromètre électronique conforme à l'invention qui est essentiellement représenté aux Figures 1 et 2, comprend généralement cinq parties :

– un portique-support 20 conçu à l'aide de profilés en aluminium et présentant une résistance mécanique élevée,

– un ensemble robotisé dit "CHARLYROBOT" qui est commercialisés en particulier par la Société CMS de Montchanin (France). Il s'agit d'un dispositif à avance linéaire qui comprend : un moteur pas à pas 5 agissant sur une vis sans fin 4 par l'intermédiaire d'un motoréducteur (non représenté) à raison de 200 pas entiers ou 400 demi-pas par tour moteur (angle de rotation de 1,8 degré par pas) ; une vis sans fin 4, couplée au motoréducteur précité, au pas de 4 mm par tour et entraînant par des vis à billes non représentées, un chariot 1 à double guidage (guidage par la vis sans fin 4 et au moyen d'un élément de guidage 1a adjacent au chariot 1 et coulissant le long d'un rail de guidage 1b qui s'étend parallèlement à la vis 4 sur toute la longueur de celle-ci) avec une précision de 0,01 mm ; un circuit électronique de pilotage 7 du moteur 5 qui comprend un micro-contrôleur de préférence du type INTEL 8051 conçu pour assurer le dialogue de ce circuit électronique 7 avec un calculateur 8 par l'intermédiaire d'un port série à 9600 bits par secondes ;

– un capteur 6 (ou seconds moyens de support) comprenant une balance de précision METTLER PM 11 permettant d'apprécier le dixième de gramme et commercialisée en particulier par les établissements Perrin à Marseille (France). Cette balance 6 repose par l'intermédiaire de plots d'amortissement sur une embase longitudinale 20a du portique support 20. Le système d'amortissement du plateau de la balance a, dans le cas de l'invention, été remplacé par un plan rigide 6a ou plan de travail reposant directement sur le capteur. La balance 6 est munie d'un port série 11 (de type RS 232) qui est conçu pour adresser au calculateur 8 les valeurs de pesée après stabilisation ainsi que d'une sortie analogique 12 dont la précision, la linéarité et le temps de réponse ont été testés,

– un jeu de câbles blindés avec filtres électroniques incorporés pour relier l'ordinateur et la balance,

– une carte électronique d'interface (non représentée) au bus PC-AT qui est conçue pour assurer la conversion analogique-digitale ou numérique de la tension de sortie analogique de la balance 6 avec une précision de 14 bits. Son temps de conversion est égal à 42 micro-secondes,

– un calculateur classique du type IBM AT et ses périphériques associés, muni en outre de deux cartes série. Dans le cas de l'invention, le calculateur utilisé est un calculateur HP Vectra ES/12.

Le dispositif comprend également une tige de mesure 2 dont une extrémité 2a est conçue pour supporter un embout de travail 3 destiné à venir en contact avec le produit 10. La tige 2 qui s'étend parallèlement à la vis sans fin 4 mais de manière décalée par rapport à celle-ci, est une tige verticale en aluminium de diamètre égal à 12 mm. Elle est fixée sur le chariot 1 par l'intermédiaire d'une équerre comportant écrou et contre-écrou pour réglage fin de la hauteur et d'un renvoi d'angle "Labomeca" du type "pied 2c" et "noix 2b". A l'extrémité inférieure 2a de la tige 2 est fixé un petit mandrin perceuse qui reçoit les embouts de travail 3, de manière amovible.

Les embouts de travail utilisés 3 (voir en particulier Figures 4A, 4B et 5) comprennent essentiellement un culot de fixation standard au mandrin de perceuse 2a précité et une tige de pénétration de forme et dimensions variées : cela peut être un cylindre de diamètre de 1 à 13 mm, à base plate ou conique. Plusieurs embouts particuliers, terminés par un plateau horizontal d'un diamètre de 50 mm environ ou par une tige horizontale permettent de réaliser des écrasements. Plusieurs coupelles ou berceaux de support 6b (Figure 3) réalisés en aluminium permettent de positionner le produit 10 sur le plan de travail 6a de la balance 6 grâce à leur courbure (traits interrompus de la Figure 3) adaptée à la taille de chaque produit 10.

Un organe spécial de travail qui est représenté à la Figure 5 est particulièrement prévu pour permettre l'arrachement des pédoncules, par exemple de la cerise. Il comprend essentiellement une tige 3a conçue pour se fixer à la tige de mesure 2, et à l'extrémité inférieure de laquelle est fixée, perpendiculairement à celle-ci, une paire de pinces "crocodile" d'électronicien qui permettent l'arrachement, particulièrement utilisable pour des pédoncules d'une cerise qui est placée sur un organe de support 6c en fer en "U" dans l'évidement duquel s'étend le pédoncule de la cerise et qui s'ouvre vers les pinces crocodiles précitées.

Le réglage de la tige de mesure 2, la longueur des embouts de travail 3, ainsi que le diamètre de leur partie fixée dans les mandrins, l'altitude du fond des organes de support 6b de chaque produit 10 sont rigoureusement identiques et intégrés dans le logiciel de fonctionnement du calculateur 8 que l'on va maintenant décrire.

Le logiciel précité est écrit en langage C (Borland International Californie Etats-Unis) C'est un logiciel qui utilise les fonctionnalités du DOS mais qui fait appel également de façon très intensive au BIOS (Basit Input Output System) pour, gérer les communications COM1 et COM2 (port série n°1, port série n°2), respectivement à 2400 et 9600 bauds selon les formats fournis par les constructeurs de la balance 6 et du "Charlyrobot ". Un circuit d'interface matériel d'acquisition de données analogiques est prévu pour détecter de façon très rapide toute variation de pesée. Son temps de conversion est inférieur à 50 micro secondes et sa précision est égale à 14 bits (16384 niveaux). La tension d'entrée de ce circuit d'interface peut varier de 0 à 8,5 volts. La tension de sortie V de la balance Mettler 6 est liée à la masse M d'un objet 10 par la relation :

$$V = 1,16\,M + 2,84$$

dans laquelle V s'exprime en volts et M en kilos, avec un coefficient de corrélation égal à 0,999.

Le logiciel précité comprend essentiellement un sous-programme utilitaire comprenant des drivers (programmes de commande) de gestion des communications COM1 et COM2 (ouverture, initialisation, synchronisation), un driver d'acquisition des tensions analogiques de pesée et des sous-programmes de saisie et de stockage des conditions de travail, c'est-à-dire opérateur, dossier, espèce, essai, fichier et série ; et un programme principal pour les différents types de mesure envisagés, c'est-à-dire :

– la mensuration.

Le sous-programme correspondant comprend les étapes consistant à descendre l'embout 3 jusqu'à détection par le port analogique 12 du capteur 6 d'une tension différentielle correspondant à la présence d'un produit 10 sur le capteur ou balance 6, déduire à partir du nombre de pas parcourus la hauteur du produit 10, faire l'acquisition de la masse du produit 10 par voie série, stocker ces paramètres de mesure sur un fichier disque, et sur ce même fichier, le numéro de l'échantillon et le nom de la série à laquelle il appartient, et répéter les étapes précédentes à la demande de l'opérateur.

Selon son désir et l'embout utilisé, l'opérateur réalisera une pénétration ou un écrasement.

Le sous-programme correspondant comprend les étapes consistant à saisir la profondeur de pénétration p souhaitée par l'opérateur soit en valeur fixée (de 0 à 60 mm), soit en % de la hauteur des produits, choisir la vitesse de pénétration, répéter les étapes de programmation correspondant à la mensuration et au stockage, déduire de la profondeur souhaitée le nombre de pas moteur qu'il y a à parcourir (n = 100 x p), exécuter l'ordre et stocker la force maximale mise en oeuvre, sa profondeur d'obtention ainsi que 10 points intermédiaires, le numéro d'échantillon et le nom de la série à laquelle il appartient, répéter ces étapes à la demande de l'opérateur ;

– l'écartement :

Le sous-programme correspondant comprend les étapes consistant à saisir le type d'écrasement voulu, c'est-à-dire le pourcentage de hauteur du produit (de 0 à 50%) et la masse à appliquer en grammes, et à répéter les étapes de programmation correspondant à la mensuration et au stockage. Selon l'objectif visé, ce sous-programme comprenant également les étapes consistant à, dans le cas d'un écrasement d'une valeur fixée ou d'une valeur proportionnelle à la hauteur du produit, déduire le nombre de pas moteur, exécuter l'ordre et stocker la force maximale, sa profondeur d'obtention ainsi que 10 points intermédiaires que le numéro de l'échantillon et le nom de la série à laquelle il appartient, et dans le cas d'une application d'une force prédéterminée, descendre progressivement l'embout 3 Jusqu'à obtention de la force demandée et calculer la hauteur parcourue d'après le nombre de pas moteur, stocker les résultats de la mesure, le numéro de l'échantillon et le nom de la série à laquelle il appartient, et répéter les étapes précitées à la demande de l'opérateur.

Un sous-programme écrasement-relaxation est également prévu selon l'invention et comprend une étape de stockage de la différence des valeurs recueillies après un temps de 2 secondes.

Le sous-programme correspondant à l'arrachement des pédoncules de cerise comprend les étapes consistant à monter le dispositif d'arrachement 3a, 3b, 6c, pincer le pédoncule, descendre le dispositif jusqu'à obtention de l'arrachement et stocker la force maximale mise en oeuvre ainsi que le numéro d'échantillon et le nom de la série à laquelle il appartient, et répéter les étapes précitées à la demande de l'opérateur.

Après chaque série de mesures, les données peuvent être inscrites sur une imprimante et sont aisément transférables sur un tableur du type Multiplan pour diverses utilisations ultérieures.

Pour apprécier la précision du pénétromètre selon l'invention, des séries de mesures ont été effectuées avec ce pénétromètre sur une sphère métallique, de préférence un boule de pétanque, ainsi que sur une balle de tennis que l'on a étudiée dans sa zone d'élasticité.

Pour tout d'abord illustrer la justesse de la mesure, 20 mesures de référence ont été effectuées sur les deux objets précités à l'aide d'une balance 6 au dixième de gramme pour la masse M et d'un pied à coulisse pour la mesure de la hauteur H. Les mêmes mesures ont été réalisées à l'aide du pénétromètre électronique. Les moyennes suivantes ont été obtenues :

| | Mesures de référence | Mesures P.E. en 1 point | en plusieurs points |
|---|---|---|---|
| M boule de de pétanque | 722,1 g | 722,1 g | 722,1 g |
| M boule de pétanque | 75,12 mm | 75,20 mm | 75,28 mm |
| M balle de tennis | 57,2 g | 57,2 | 57,2 g |
| M balle de tennis | 64,14 mm | 64,18 | 64,09 mm |

Dans ce tableau, P.E. signifie pénétromètre électroniques.

Les éléments constitutifs du pénétromètre ont leur sensibilité propre :

– La balance Mettler 6 apprécie le 1/10 de g.

– Le Charlyrobot contrôle le 1/2 pas, soit un angle de rotation de 0,9°, compte-tenu du motoréducteur.

– La vis sans fin 4 au pas de 4 mm nécessite 400 demi-pas pour effectuer 1 tour, soit une sensibilité de :

$$\frac{4}{400} = 0,52 \text{ mV}$$

– La carte de sortie analogique permet d'acquérir une tension maximum de 8,5 v avec une précision de 14 bits. La plus petite tension perceptible est donc :

$$\frac{8,5 \text{ V}}{2^{14}} = \frac{8,5 \text{ V}}{16\,384} = 0,52 \text{ mV}$$

Par ailleurs, en se reportant à la relation liant V et M précitée, on obtient :

$$V = 1,16 \text{ M} + 2,84$$

$$M = \frac{8,5 - 2,84}{1,6} = 4,87 \text{ kg utilisables}$$

La plus petite masse décelable est donc :

$$\frac{m}{0,52.10^{-3}} = \frac{4,87.10^3}{8,5 - 2,84}$$

$$m = 0,45 \text{ g}$$

Il est possible en utilisant un câble spécifique et une version adaptée du logiciel d'obtenir une plage de mesure double. Dans ce cas là, la plus petite masse décelable est m = 1g.

En se reportant aux deux expériences précédentes, on a noté avec le pénétromètre une parfaite concordance des mesures de poids et de hauteur lorsque les objets restent immobiles. Lorsque la position des objets est modifiée à chaque mesure par une rotation aléatoire sur son socle, on note toujours le même poids mais des variations interviennent dans la hauteur, montrant leur irrégularité :

M boule de pétanque =75,28 mm Ecart type

sigma =0,07

M balle de tennis= 64,09 mm Ecart type

sigma=0,15

Au seuil de 1 % avec n = 20, le texte de STUDENT FISCHER donne :

M boule de pétanque = 75,28 +/-0,2 mm soit 5,3%o

M balle de tennis = 64,09+/- 0,43 mm soit 1,2 %

Des essais d'écrasement d'une balle de tennis de hauteur 67,4 mm ont été réalisés par série de 13 sans modification de la position de l'objet. On a obtenu successivement pour 2, 3 et 4 kg les déplacements suivants:

| F kg | d H mm | sigma | test t |
|------|--------|-------|--------|
| 2 | 2,56 | 0,06 | +/- 0,15 |
| 3 | 3,42 | 0,04 | +/- 0,12 |
| 4 | 4,38 | 0,04 | +/- 0,12 |

Ces résultats montrent la précision du pénétromètre selon l'invention.

Les produits vivants et en particulier les fruits et légumes sont sujets à une grande variabilité biologique en fonction des facteurs qui ne sont pas toujours contrôlés : ensoleillement, position sur la plante, état réel de maturité, calibre, climat, etc. Il importe donc que les méthodes de mesure ne viennent pas ajouter d'incertitude supplémentaire dans les résultats et qu'elles soient adaptées aux produits.

La géométrie du pénétromètre permet de travailler sur des espèces de tailles aussi différentes que cerise, pêche, melon, etc. De même, la précision des mesures dans une large gamme de valeurs permet de travailler sur des produits mous tels que les fraises comme sur des produits fermes, pommes ou aubergines par exemple.

La pénétration est la méthode la plus couramment utilisée sur les produits frais. Selon l'état du produit, on utilise des embouts 3 de diamètre variable. La valeur recueillie avec les appareils actuels est la force maximale correspondant à la rupture des cellules. L'intérêt du pénétromètre est de maîtriser parfaitement la vitesse de pénétration et en particulier d'éviter l'inertie du geste manuel lors de la rupture, de connaître avec une grande précision la valeur de la force maximale ainsi que sa profondeur d'obtention et de garder en mémoire les points permettant de tracer la courbe force pénétration. Ce type de mesure peut convenir à tous les fruits à chair homogène mais présente cependant l'inconvénient d'être destructif.

L'écrasement correspond mieux à l'impression que l'on a en tatant un produit susceptible de devenir mou. Il s'applique en priorité aux produits à chair non homogène tels que tomate, aubergine, poivron, ou à chair molle dans une enveloppe résistante comme la cerise ou le raison. La précision de l'appareil permet également de mesurer des micro-déformations par incréments de 1/100 mm. Cette méthode a le grand avantage d'être non destructive.

Plusieurs tests sont proposés :

a) Ecrasement sous une force donnée, avec recueil du déplacement obtenu.

A titre d'exemple, une force de 3,5 kg a provoqué un écrasement de 1,5 à 4,5 mm sur melon selon son état et sa maturité.

b) Ecrasement en % du diamètre du produit avec recueil de la force maximum obtenue.

On peut imaginer qu'une force constante ou qu'un écrasement d'une hauteur constante pénalise les produits selon leur calibre surtout s'il y a présence d'un noyau important. C'est pourquoi la possibilité est offerte de proportionner la contrainte à la taille mesurée (0 à 50%).

A titre d'exemple, on a recueilli des forces moyennes de 1 054 g pour écraser de 5% des tomates rouges et de 1 475 g pour une même opération sur un lot de tomates vertes tournant au rouge. Deux variétés de cerises mûres écrasées de 10% de leur diamètre ont nécessité respectivement 258 et 385 g. Un test de mesures rhéologiques est aussi prévu pour contrôler la perte d'élasticité des produits entre deux écrasements.

c) Ecrasement sur une hauteur fixée. Des essais conduits sur 17 variétés d'abricots, chacune étant caractéristique de trois stades différents de maturité et chaque stade étant subdivisé en trois lots successivement testés le jour même, 4 jours puis 7 jours après cueillette, ont permis d'établir une échelle de fermeté de ces variétés selon le stade de cueillette et le temps de concentration.

Le support spécial 6c et l'embout à pinces 3a, 3b permettent de mesurer la force d'arrachement des pédoncules de cerise. Cette application est intéressante lors des contrôles de vieillissement et pour mesurer l'aptitude des cerises à la récolte mécanique.

Le pénétromètre étant au départ un appareil de laboratoire, il a paru intéressant d'allier le poids et les mensurations des produits aux mesures de fermeté. Il est possible de n'effectuer que les mensurations (pesée et diamètre) qui peuvent par exemple être utilisées en pomologie. Ces mesures sont intégrées aux séquences programmées de fermeté-pénétration et fermeté-écrasement de façon à vérifier s'il existe des interactions calibre-fermeté dans les lots testés.

Toutes les données obtenues sont stockées en ASCII sur fichier, soit sur un disque dur, soit sur une disquette. Elles sont facilement transférables sur des tableurs notamment du type de celui commercialisé sous la dénomination MULTIPLAN.. Elles peuvent donc être corrélées à d'autres observations et traitées sur des logiciels statistiques, tel que celui commercialisé sous la dénomination STATITEF sans nouvelle saisie. L'on peut par exemple établir des moyennes, écarts-types, pertes en différents points des courbes, connaître l'abscisse des valeurs maximum etc et en déduire des comparaisons entre différents lots de produits.

D'autres essais ont été réalisés.

Les essais précités montrent que le pénétromètre électronique conforme à l'invention est un appareil précis, fidèle et robuste. Les possibilités de mesure et d'évaluation en matière de perte de fermeté associées à la pesée et à la mensuration des produits en font un instrument précieux pour la technologie des fruits et des légumes ou pour tout autre produit solide comparable devant faire l'objet de mesures précises de fermeté ou d'écrasement. Son logiciel permet d'adapter toutes sortes de paramètres à l'espèce étudiée.

Des études plus détaillées peuvent être entreprises avec cet appareil en toute confiance car ses performances ne sont pas critiquables. Cela est extrêmement important lorsqu'on connait la variabilité biologique des fruits et des légumes. Finalement, le coût du pénétromètre conforme à l'invention est très modéré et le rend par conséquent très compétitif.

Les signes de référence insérés après les caractéristiques techniques mentionnées dans les revendications, ont pour seul but de faciliter la compréhension de ces dernières et n'en limitent aucunement la portée.

## Revendications

1. Dispositif pour déterminer automatiquement des caractéristiques physiques telles que les caractéristiques dimensionnelles et/ou la fermeté d'un produit (10), notamment un fruit ou un légume, caractérisé par des premiers moyens supports (1) d'une tige de mesure (2) dont une extrémité (2a) est conçue pour supporter un embout de travail (3) destiné à venir en contact avec le produit (10), lesdits moyens supports (1) de la tige de mesure (2) étant montés à translation par rapport à une vis sans fin (4) qui s'étend sensiblement parallèlement à la tige de mesure (2) et qui est entraînée en rotation par des moyens moteurs (5) du dispositif, et en ce que le produit (10) est disposé sur des seconds moyens supports (6) formant capteur de mesures dont le plan de travail (6a) s'étend sensiblement perpendiculairement à la tige de mesure (2), les moyens (6) formant capteur de mesure étant reliés à des moyens informations (8) du type ordinateur ou analogique qui sont également reliés à des moyens de commande électroniques (7) du moteur (5), les moyens informatiques (8) étant programmés pour traiter et stocker les mesures réalisées par le dispositif.

2. Dispositif selon la revendication 1, caractérisé en ce que les moyens moteurs (5) sont reliés aux moyens informatiques (8) par l'intermédiaire d'un circuit électronique de pilotage (7) à micro-contrôleur qui réalise le dialogue avec les moyens informatiques (8).

3. Dispositif selon la revendication 2, caractérisé en ce que le dialogue entre le circuit de pilotage (7) et les moyens informatiques (8) est réalisé par l'intermédiaire d'un port série à 9600 bits/s du circuit de pilotage (7).

**4.** Dispositif selon l'une des revendications précédentes, caractérisé en ce que la tension de sortie analogique délivrée par les moyens capteurs (6) est appliquée aux moyens informatiques (8) par l'intermédiaire d'un circuit d'interface conçu pour assurer une conversion analogique-digitale de cette tension de sortie et pour détecter rapidement toute variation de mesure.

**5.** Dispositif selon l'une des revendications précédentes, caractérisé en ce que les moyens moteurs comprennent un moteur pas à pas (5) dont l'arbre de sortie est couplé à la vis sans fin (4) par l'intermédiaire d'un motoréducteur qui produit une avance d'environ 200 pas par tour.

**6.** Dispositif selon l'une des revendications précédentes, caractérisé en ce que les moyens formant capteur comprennent une balance de précision (6) de type Mettler qui présente une sensibilité d'environ 0,1 g et qui possède un port série (11) ainsi qu'une sortie analogique (12) reliés aux moyens informatiques (8).

**7.** Dispositif selon l'une des revendications précédentes, caractérisé en ce que les moyens moyens supports comprennent un chariot (7) monté sur un portique-support (20) par l'intermédiaire d'un dispositif à double guidage et entraîné par la vis sans fin (4) par l'intermédiaire de vis à billes.

**8.** Dispositif selon l'une des revendications précédentes, caractérisé en ce que l'embout de travail (3) comporte, du côté de la tige de mesure (2), un culot de fixation amovible à l'extrémité (2a) de cette tige de mesure (2) qui est conformée en mandrin de perceuse et, du côté opposé, une tige de pénétration du produit (10) ou un plateau d'écrasement de celui-ci.

**9.** Dispositif selon l'une des revendications précédentes, caractérisé par au moins un support en aluminium (6b) de positionnement du produit (10) sur le plan de travail (6a) des moyens formant capteur (6).

**10.** Procédé de mesure mis en oeuvre au moyen d'un dispositif selon l'une des revendications précédentes, caractérisé par les étapes consistant à :
a) descendre l'embout de travail (3) jusqu'à détection par le port analogique (11) des moyens capteurs (6) d'une tension différentielle indicative de la présence d'un produit (10) sur le plan de travail (6a) des moyens capteurs,
b) déduire, à partir du nombre de tours effectués par les moyens moteurs (5), la hauteur du produit (10),
c) faire l'acquisition de la masse du produit (10) au moyen de la liaison série entre les moyens capteurs (6) et les moyens informatiques (8),
d) stocker les paramètres de mesures précités sur un fichier disque et, sur ce même fichier, les références de l'échantillon de produit.

**11.** Procédé selon la revendication 10, caractérisé par les étapes complémentaires consistant à :
– saisir la profondeur de pénétration (p) du produit souhaité, soit en valeur fixée, soit en pourcentage de la hauteur du produit,
– choisir la vitesse de pénétration,
– répéter les étapes a) à d) précitées,
– déduire de la profondeur (p) souhaitée, le nombre (n) de pas moteur à parcourir,
– exécuter l'ordre de mesure,
– stocker la force maximale appliquée et la profondeur d'obtention de cette force,
– stocker les points intermédiaires permettant de tracer la courbe de force de pénétration,
– répéter si nécessaire les étapes précédentes.

**12.** Procédé selon la revendication 10, caractérisé par les étapes complémentaires consistant à :
– saisir le type d'écrasement souhaité, en profondeur d'écrasement en pourcentage de hauteur du produit (10) ou en masse à appliquer,
– répéter les étapes a) à d) précitées,
– en déduire le nombre de pas moteur, exécuter l'ordre de mesure, stocker la force maximale appliquée et sa profondeur d'obtention, stocker les points intermédiaires permettant de tracer la courbe force/déplacement,
– ou calculer la hauteur parcourue par l'embout (3) d'après le nombre de pas moteur jusqu'à obtention de la masse fixée et stocker cette hauteur,
– répéter si nécessaire les étapes précitées.

FIG.2

FIG.1

FIG.3

FIG.4A   FIG4B

FIG.5

EP 0 439 405 A1

Office européen
des brevets

**RAPPORT DE RECHERCHE EUROPEENNE**

Numero de la demande

EP 91 40 0150

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FR-A-2 595 824 (INSTITUT NATIONAL DE LA RECHERCHE AGRONOMIQUE) * Page 4, lignes 6-30; page 5, ligne 4 - page 6, ligne 15; page 6, lignes 32-36; page 8, lignes 6-9; figures 1-3 * | 1-8 | G 01 N 3/42 G 01 N 33/02 G 01 B 3/00 |
| A | --- | 10-12 | |
| Y | DE-A-3 219 766 (ELESTA AG ELEKTRONIK) * Figure 1; pages 9-11 * | 1-8 | |
| A | --- | 10 | |
| A | US-A-4 061 020 (R.B. FRIDLEY et al.) * Résumé; colonne 2, lignes 1-10,32-42,53-64; colonne 3, lignes 24-42 * | 10-11 | |
| A | --- GB-A-1 228 774 (RUBBER AND PLASTIC RESEARCH ASSOCIATION OF GB) * Page 1, lignes 20-74; figure 1 * | 10 | |
| A | --- FR-A-1 600 614 (SOCIETE D'APPLICATIONS GENERALES D'ELECTRICITE ET DE MECANIQUE) * Page 2, lignes 1-7,33-36; figure 3 * ----- | 5,7 | DOMAINES TECHNIQUES RECHERCHES (Int. Cl.5) G 01 N G 01 B |

Le présent rapport a été établi pour toutes les revendications

| Lieu de la recherche | Date d'achèvement de la recherche | Examinateur |
|---|---|---|
| LA HAYE | 25-04-1991 | HOCQUET A.P.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)